# EUROPEAN PATENT APPLICATION

(11) **EP 4 368 119 A1**
(43) Date of publication of application: **15.05.2024**
(21) Application number: 21925100.6
(22) Date of filing: 24.08.2021
(51) Int. Cl.: A61B 10/00

(54) **QUANTITATIVE SALIVA SAMPLE COLLECTION TOOL**

(30) Priority: 06.07.2021 KR 20210088655
(71) Applicant: Noblebio Co., Ltd., Hwaseoug-si, Gyeonggi-do 18521 (KR); Noble M&B Co., Ltd., Yongin-si, Gyeonggi-do 17118 (KR)
(72) Inventor: BAEK, Kye Seung, Suwon-si Gyeonggi-do 16383 (KR); LEE, Joo Hoon, Yongin-si Gyeonggi-do 17056 (KR); HWANG, Chang Nam, Seoul 04724 (KR)
(74) Representative: Gulde & Partner
(86) International application number: PCT/KR2021/011271
(87) International publication number: WO 2023/282383

(57) **Abstract**

Provided is a quantitative saliva specimen collection tool, and more particularly to, a quantitative saliva specimen collection tool in which a plurality of collection protrusions are formed to protrude on both surfaces of a header part of a swab portion, and the plurality of collection protrusions are formed to be spaced apart from each other at a predetermined interval, when a saliva specimen is collected, the saliva specimen is collected by minimizing the volume of saliva between the collection protrusions so that only a quantitative amount of saliva is collected to the extent that the saliva does not from flow from the header part, the header part on which the saliva is collected is broken through a breaking groove of a stick part and sealed and stored in a tube portion, and then, a rapid immunological diagnosis or an analysis that requires an appropriate amount in the field, such as a screening clinic, or a molecular diagnosis and immunology rapid diagnosis by transporting the header part to a laboratory may be tested accurately and safely.

## Description

### TECHNICAL FIELD

The present invention relates to a quantitative saliva specimen collection tool, and more particularly to, a quantitative saliva specimen collection tool in which a plurality of collection protrusions are formed to protrude on both surfaces of a header part of a swab portion, and the plurality of collection protrusions are formed to be spaced apart from each other at a predetermined interval, when a saliva specimen is collected, the saliva specimen is collected by minimizing the volume of saliva between the collection protrusions so that only a quantitative amount of saliva is collected to the extent that the saliva does not from flow from the header part, the header part on which the saliva is collected is broken through a breaking groove of a stick part and sealed and stored in a tube portion, and then, a rapid immunological diagnosis or an analysis that requires an appropriate amount in the field, such as a screening clinic, or a molecular diagnosis and immunology rapid diagnosis by transporting the header part to a laboratory may be tested accurately and safely.

### BACKGROUND ART

In general, with the development of medicine and various related technologies, substances such as blood cells, nucleic acids, proteins and antigens, antibodies and drugs included in a predetermined biological sample such as saliva, blood, urine, etc. are tested. After collecting the sample as described above, by analyzing and observing changes that occur after reacting the collected sample with a predetermined reagent, the presence or absence of various substances included in the sample, ratio and amount, etc. may be tested, and accordingly, it is possible to obtain information about the presence or absence of a disease, the state of the disease, etc.

In a test process of such a sample, it is important to prevent contamination of the sample due to external exposure not only in a process of collecting the sample, but also in a process of storing and inputting the collected sample. In addition, in the process of collecting saliva, convenience is required in a process of collecting samples such as children, the elderly, or animals with poor cognitive ability.

For example, as in Korea Patent Registration No. 10-1927053, when human saliva is collected, stored, or used, contamination may occur in a process of collecting saliva, and, a process of transferring the collected saliva may be necessary to a separate storage device to store the collected saliva. In addition, even when using the collected saliva, there is a case where a separate device is required. In addition, it is difficult to collect saliva of children and the elderly with communication difficulties and animals in the process of collecting the saliva.

Accordingly, there is a need to develop a device capable of easily collecting, storing and using samples such as saliva from people or animals, such as children, the elderly, and animals.

### DESCRIPTION OF EMBODIMENTS

### TECHNICAL PROBLEM

Provided is a quantitative saliva specimen collection tool in which a plurality of collection protrusions are formed to protrude on both surfaces of a header part of a swab portion, and the plurality of collection protrusions are formed to be spaced apart from each other at a predetermined interval, when a saliva specimen is collected, the saliva specimen is collected by minimizing the volume of saliva between the collection protrusions so that only a quantitative amount of saliva is collected to the extent that the saliva does not from flow from the header part, the header part on which the saliva is collected is broken through a breaking groove of a stick part and sealed and stored in a tube portion, and then, a rapid immunological diagnosis or an analysis that requires an appropriate amount in the field, such as a screening clinic, or a molecular diagnosis and immunology rapid diagnosis by transporting the header part to a laboratory may be tested accurately and safely.

### SOLUTION TO PROBLEM

According to an aspect of the present invention, a quantitative saliva specimen collection tool includes a swab portion including a head part to directly collect saliva, and a stick part integrally formed on one side of the head part to support the head part, and a tube portion transported after storing the swap portion inside and sealing the inside with a lid.

A plurality of collection protrusions may be formed to protrude from one side of the head part and are spaced apart from each other so that saliva is quantitatively filled between the collection protrusions, and the plurality of collection protrusions may be formed to protrude apart from each other on the other side of the head part so that saliva is quantitatively filled between the collection protrusion and the collection protrusion.

A breaking groove in which a breaking point is formed may be formed in one end of the stick part so that when the swab portion is stored inside the tube portion, only the head part is separated and inserted into the tube portion.

### ADVANTAGEOUS EFFECTS OF DISCLOSURE

As described above, the quantitative saliva specimen collection tool of the present invention in which a plurality of collection protrusions are formed to protrude on both surfaces of a header part of a swab portion, and the plurality of collection protrusions are formed to be spaced apart from each other at a predetermined interval, when a saliva specimen is collected, the saliva specimen is collected by minimizing the volume of saliva between the collection protrusions so that only a quantitative amount of saliva is collected to the extent that the saliva does not from flow from the header part, the header part on which the saliva is collected is broken through a breaking groove of a stick part and sealed and stored in a tube portion, and then, a rapid immunological diagnosis or an analysis that requires an appropriate amount in the field, such as a screening clinic, or a molecular diagnosis and immunology rapid diagnosis by transporting the header part to a laboratory may be tested accurately and safely.

### BRIEF DESCRIPTION OF DRAWINGS

FIG. 1 is an exploded perspective view showing a quantitative saliva specimen collection tool according to an embodiment of the present invention.
FIG. 2 is a cross-sectional view showing a quantitative saliva specimen collection tool according to an embodiment of the present invention.
FIG. 3 is a front view showing a swab portion according to an embodiment of the present invention.
FIG. 4 is a plan view showing a swab portion according to an embodiment of the present invention.
FIG. 5 is a cross-sectional view showing a swab portion according to an embodiment of the present invention.

### BEST MODE

The present invention having such features may be more clearly described through preferred embodiments according thereto.

Before describing various embodiments of the present invention in detail with reference to the accompanying drawings, it will be appreciated that the application is not limited to the details of configurations and arrangements of elements described in the following detailed description or illustrated in the drawings. The present invention may be implemented and practiced in different embodiments, and may be performed in various ways. Also, it will be appreciated that the expressions and predicates used herein with respect to terms such as the device or element orientation (e.g. "front", "back", "up", "down", "top", "bottom", "left", "right", and "lateral"), etc. are used only to simplify the description of the present invention, and related device or element does not indicate or mean simply a specific orientation. Further, terms such as "first" and "second" are used in this application and the appended claims for purposes of explanation and are not intended to represent or imply any relative importance or spirit.

Accordingly, the embodiments described in the present specification and the configurations shown in the drawings are only the most preferred embodiments of the present invention, and do not represent all the technical ideas of the present invention, it should be understood that there may be various equivalents and variation that may replace these at the time of filing the present application.

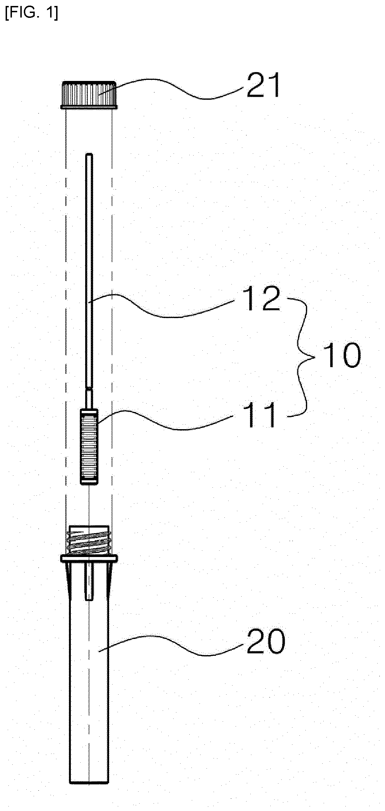

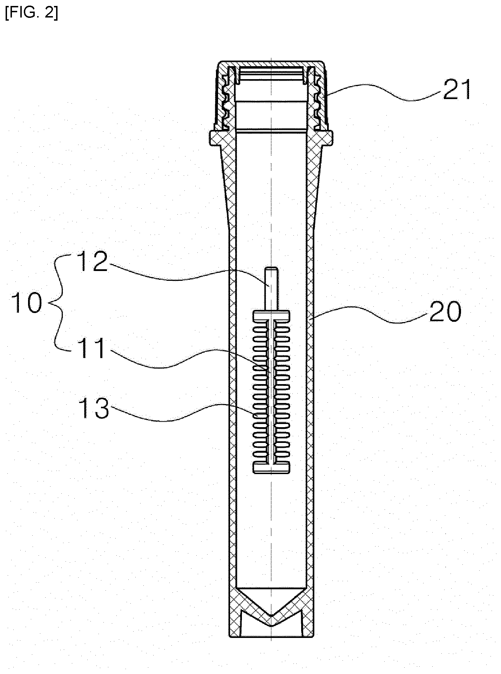

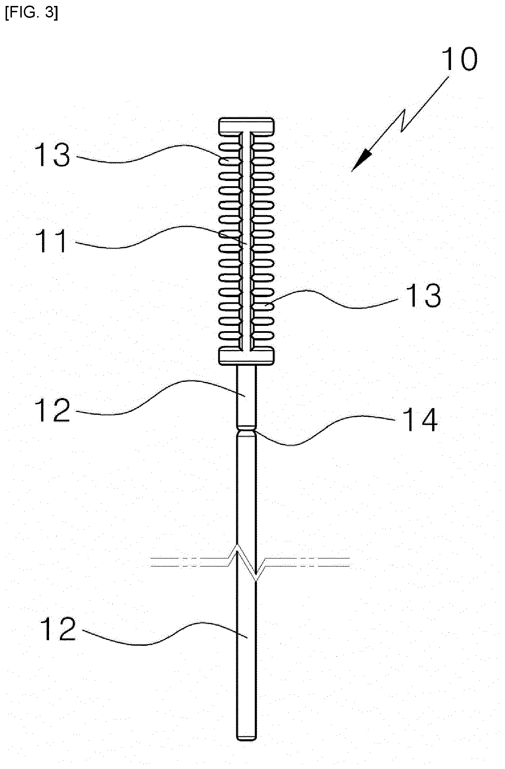

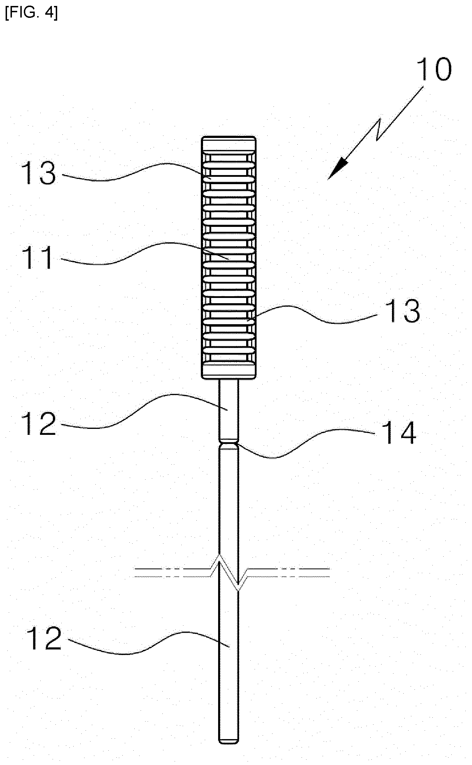

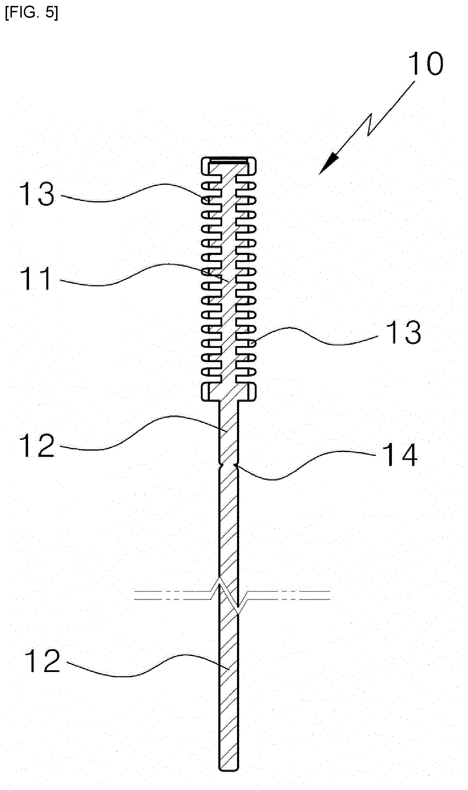

FIG. 1 is an exploded perspective view showing a quantitative saliva specimen collection tool according to an embodiment of the present invention. FIG. 2 is a cross-sectional view showing the quantitative saliva specimen collection tool according to an embodiment of the present invention. FIG. 3 is a front view showing a swab portion according to an embodiment of the present invention. FIG. 4 is a plan view showing the swab portion according to an embodiment of the present invention. FIG. 5 is a cross-sectional view showing the swab portion according to an embodiment of the present invention.

As shown in FIGS. 1 to 5, the quantitative saliva specimen collection tool of the present invention is a device for collecting a certain amount (quantitative) of saliva samples for molecular diagnosis and rapid immunology diagnosis, and includes a swab portion 10 and a tube portion 20.

As shown in FIGS. 1 to 5, the swap portion 10 includes a head part 11 for directly collecting saliva and a stick part 12 integrally formed on one side of the head part 11 to support the head part 11.

Here, the head part 11 is integrally formed on an end of the stick part 12, a plurality of collection protrusions 13 are formed to protrude from one side of the head 11, and the plurality of collection protrusions 13 are formed to be spaced apart from each other at a predetermined interval.

Also, the plurality of collection protrusions 13 are formed to protrude apart from each other on the other side of the head part 11, so that saliva is collected between the collection protrusion 13 and the collection protrusion 13 quantitatively. That is, an amount of saliva collection is determined according to an interval between the collection projection 13 and the collection projection 13, and in the present invention, the volume of drops of saliva between the collection projection 13 and the collection projection 13 is minimized so that an amount of saliva specimen collection is collected so as not to flow the saliva down from the head part 11 as much as possible. At this time, the interval between the collection protrusion 13 and the collection protrusion 13 is formed as 0.1 - 2 mm.

In addition, the collection protrusion 13 is formed to protrude vertically from one surface of the head part 11, and is formed in the shape in which a width becomes narrower toward an upper side, that is, in a narrow upper and wide lower shape, and the end of the collection protrusion 13 is formed to be spherical so that the saliva is easily introduced between the collection protrusion 13 and the collection protrusion 13.

On the other hand, the stick part 12 is integrally formed to protrude from one end of the head part 11, and is used to collect the saliva through the head part 11 by directly holding the stick part 12 by a user.

Here, a breaking groove 14 in which a breaking point is formed is formed in one end of the stick part 12 so that when the swab portion 10 is stored inside the tube portion 20, only the head part 11 is separated and inserted into the tube portion 20, and the breaking groove 14 is formed along an outer periphery of the stick part 12 so that one end of the stick part 12 may be easily broken.

As such, an appropriate amount of saliva collection of the swab portion 10 of the present invention is different depending on the size of the head part 11. As an embodiment, when the size of the head part 11 is 34 mm, the saliva of about 200 ul is collected, when the size of the head part 11 is 28mm, it is possible to collect the saliva of about 150 ul, but it is not possible to limit the appropriate amount because there is a difference depending on a specimen person.

As shown in FIGS. 1 to 2, the tube portion 20 is transported after storing the swap portion 10 inside and sealing the inside with a lid 21.

Here, one side of the tube portion 20 is opened to store the head part 11 of the swap portion 10 inside the tube portion 20, the lid 21 for opening and closing the inside of the tube portion 20 is screwed to the opened one side of the tube portion 20, and the head part 11 in which one end of the stick part 12 is broken is protected by being included inside the tube portion 20, that is, the outside of the head part 11 is wrapped and protected. At this time, the tube portion 20 is in the shape of a transparent tube, and the head part 11 provided inside the tube portion 20 may be visually identified.

In addition, the tube portion 20 has a male thread formed on its outer periphery to be screwed with the lid 21, and an inner bottom surface of the tube portion 20 is formed to be inclined toward a central part in a wide upper and narrow lower shape so as to easily collect the specimen through the end of the head part 11 or to conveniently transport the tube portion 20 to a lower side.

In addition, a solution necessary for molecular diagnosis and rapid immunological diagnosis is stored inside the tube portion 20, when the head part 11 on which saliva is collected is input to the inside of the tube portion 20, the inside of the tube portion 20 is sealed with the lid 21, and then the tube portion 20 is shaken so that the solution and the saliva are well mixed.

Hereinafter, a method of using the quantitative saliva specimen collection tool described above will be described.

In the method of using the quantitative saliva sample collection tool of the present invention, as a first step of collecting a saliva specimen, first, a person to collect saliva massages both cheeks to collect enough saliva in the mouth, inserts the head part 11 of the swab portion 10 between the collected saliva, and slowly rotates the swap portion 10 so that the saliva may be sufficiently put on between the collection protrusions 13 of the head part 11.

As a second step of preparing a container (tube portion), the tube portion 20 is prepared to put the head part 11 of the swab portion 10 on which the saliva specimen is put thereinto.

As a third step of putting the head part 11 into the container (tube portion), first, the swab portion 10 on which the saliva specimen is put is carefully inserted into the tube portion 20 so that the saliva does not flow to the outside of the tube portion 20 and the breaking groove 14 of the stick part 12 is broken.

Thereafter, the lid 21 prepared in advance is locked, and then, the tube portion 20 is sufficiently shaken so that the solution and the saliva specimen may be mixed.

Then, the tube portion 20 is transported to a laboratory, an appropriate amount (1 drop/about 25 ul) is put into a device hole, identified in a planar figure, and diagnosed in a molecular and immunological rapid test.

### [Descriptions of Reference Numerals]

| | | | |
|---|---|---|---|
| 10: | swap portion | 11: | head part |
| 12: | stick part | 13: | collection protrusion |
| 14: | breaking groove | | |
| 20: | tube portion | 21: | lid |

## Claims

1. A quantitative saliva specimen collection tool comprising:
a swab portion including a head part to directly collect saliva, and a stick part integrally formed on one side of the head part to support the head part; and
a tube portion transported after storing the swap portion inside and sealing the inside with a lid.

2. The quantitative saliva specimen collection tool of claim 1,
wherein a plurality of collection protrusions are formed to protrude from one side of the head part and are spaced apart from each other so that saliva is quantitatively filled between the collection protrusions, and
wherein the plurality of collection protrusions are formed to protrude apart from each other on the other side of the head part so that saliva is quantitatively filled between the collection protrusion and the collection protrusion.

3. The quantitative saliva specimen collection tool of claim 1,
wherein a breaking groove in which a breaking point is formed is formed in one end of the stick part so that when the swab portion is stored inside the tube portion, only the head part is separated and inserted into the tube portion.
